# EUROPEAN PATENT SPECIFICATION

(11) **EP 0 778 024 B1**
(45) Date of publication and mention of the grant of the patent: **13.11.2002**
(21) Application number: 97101631.6
(22) Date of filing: 14.01.1994
(51) Int. Cl.: A61K 31/47, A61P 35/00

(54) **Oral or intramuscular treatment of pancreatic cancer with water-insoluble S-Camptothecin of the closed lactone ring form**
Orale oder intramuskuläre Behandlung von Pankreaskrebs durch wasserunlösliches S-Camptothecin mit geschlossenem Lactonring
Traitement oral ou intramusculaire du cancer pancreatique au moyen de S-Camptothecine insoluble dans l'eau de la forme fermée du cycle de lactone

(30) Priority: 15.01.1993 US 2844
(43) Date of publication of application: 11.06.1997
(62) Divisional of application: 94907804.2
(73) Proprietor: THE STEHLIN FOUNDATION FOR CANCER RESEARCH, Houston, TX 77002 (US)
(72) Inventor: Giovanella, Beppino C., Houston, Texas 77027 (US); Hinz, Hellmuth R., Kingwood, Texas 77339 (US); Kozielski, Anthony J., Springs, Texas 77379 (US); Stehlin, John S., Houston, Texas 77024 (US)
(74) Representative: Sexton, Jane Helen

(56) References cited:
- EP-A- 0 418 099
- EP-A- 0 540 099
- WO-A-91/05556
- CANCER RESEARCH, vol. 52, no. 14, 15 July 1992, pages 3980-3987, XP002028493 P. PANTAZIS ET AL.: "Complete inhibition of growth followed by death of human malignant melanoma cells in-vitro and regression of human melanoma xenografts in immunodeficient mice induced by camptothecins"

## Description

### Field of the Invention

Use of 20(S)-camptothecin and analogs thereof in the manufacture of a medicament for the treatment of pancreatic cancer by oral or intramuscular administration.

### Background of the Invention

20(S)-camptothecin (CPT), a plant alkaloid, was found to have anticancer activity in 1966 (Wall, M., Wani, M.C., Cooke, C.E., Palmer, K.H., McPhail, A.T. and Slim, G.A. "Plant antitumor agents. I. The isolation and structure of camptothecin, a novel alkaloidal leukemia and tumor inhibitor from Camptotheca acuminata", J. Am. Chem. Soc. 88: 3888-3890, 1966).

During the sixties and seventies the sodium salt of CPT was derived from CPT, and clinical trials of this chemically altered CPT were carried out and then abandoned because of the high toxicity and low potency of this compound (Gottlieb, J.A., Guarino, A.M., Call, J.B., Oliverio, V.T. and Block, J.B. "Preliminary pharmacological and clinical evaluation of camptothecin sodium salt (NSC 100880)", Cancer Chemother. Rep. 54: 461-470; 1979; Muggia, F.M., Creaven, P.J., Hansen, H.H., Cohen, M.N. and Selawry, D.S. "Phase I clinical trials of weekly and daily treatment with camptothecin (NSC 100880). Correlation with clinical studies." Cancer Chemother. Rep. 56: 515-521; 1972; Gottlieb, J.A. and Luce, J.K. "Treatment of malignant melanoma with camptothecin (NSC 100880)." Cancer Chemother. Rep. 56: 103-105; 1972; and Moertel, C.G., Schutt, A.J., Reitemeier, R.J. and Hahn, R.G. "Phase II study of camptothecin (NSC 100880) in the treatment of advanced gastrointestinal cancer." Cancer Chemother. Rep. 56: 95-101; 1972. All these trials were conducted using the hydrosoluble, sodium salt derivative of CPT (CPT Na+), which was administered via i.v. The research of the present inventors has fully confirmed the ineffectiveness and the toxicity of CPT Na+.

Experiments have demonstrated that the non-water soluble CPT is nontoxic and highly effective as an anticancer agent. Furthermore, the present inventors have demonstrated that the intramuscular and oral administration provide unexpectedly better results than the intravenous administration.

Drug therapies have been evaluated with respect to treating human cancer, e.g., human cancer xenograft lines. Human tumors are serially heterotransplanted into immunodeficient, so-called "nude" mice, and the mice then tested for their responsiveness to a specific drug. (Giovanella, B.C., et al., Cancer 52(7):1146 (1983)). The data obtained in these studies strongly support the validity of heterotransplanted human tumors into immunodeficient mammals, such as nude mice, as a predictive model for testing the effectiveness of anticancer agents.

It was determined that 9-Amino-20(S)-Camptothecin (9AC) and 10,11-Methylendioxy-20(S)-Camptothecin (10,11MD) are capable of having high anticancer activity against human colon cancer xenografts (Giovanella, B.C., Wall, M.E., Wani, M.C., Nicholas, A.W., Liu, L.F., Silber, R. and Potmesil, M. "Highly effective topoisomerase-I targeted chemotherapy of human colon cancer in xenografts." Science 246: 1046-1048; 1989). After this finding, the present inventors extended these studies to other human cancers growing as xenografts in nude mice as well as conducted studies on the administration of CPT and its derivatives. It is important to note that the fundamental difference between the chemical used by the present invention (CPT) and the one used ineffectively and with high attendant toxicity in the past (CPT Na+) is that CPT is water-insoluble and CPT Na+ is water-soluble.

### Summary of the Invention

An object of the present invention is to provide a novel medicament for the treatment of pancreatic cancer in a human.

Additional, objects and advantages of the present invention will be set forth in part in the description which follows, and in part will be apparent from the description, or may be learned by practice of the present invention The objects and advantages of the present invention will be realized and attained by means of the elements and combinations particularly pointed out in the appended claims.

To achieve the objects and in accordance with the purpose of the present invention, as embodied and broadly described herein, the present invention relates to use of an agent which is 20(S)-camptothecin, 9-Nitro-20(S)-camptothecin, 9-Amino-20(S)-camptothecin, or a mixture thereof, in the manufacture of a medicament for the treatment of pancreatic cancer in a human comprising oral or intramuscular administration of an effective amount of said agent.

It is to be understood that both the foregoing general description and the following detailed description are exemplary and explanatory only and are not restrictive of the present invention, as claimed.

### Brief Description of the Drawings

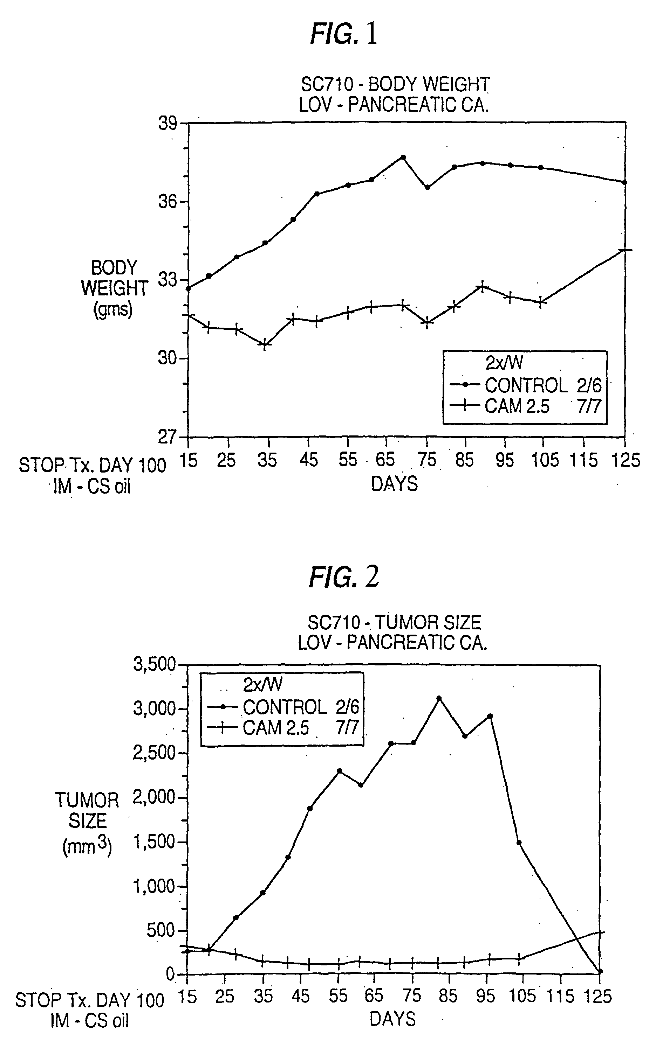

Figures 1 and 2 are graphs which show the results obtained for LOV pancreatic carcinoma in nude mice treated twice a week with 20(S)-CPT in cottonseed oil administered intramuscularly at the dose of 2.5 mg/Kg.

### Description of the Preferred Embodiment(s)

CPT is a plant alkaloid isolated from Camptotheca acuminata. Chemical derivatives of CPT can be prepared either in a semisynthetic or totally synthetic way. (See, e.g., Wani, M.C. et al., J. Med. Chem. 23:544, 1980; Wani, M.C. et al., J. Med. Chem. 30: 1774 (1987); and Wani, M.C. et al., J. Med. Chem. 30: 2317 (1987)).

In the examples discussed below, the CPT was obtained from the Institute of Materia Medica, Academia Sinica, Shanghai, China, and further purified using standard chromatographic techniques. Camptothecin Sodium Salt (CPT Na+), 9-Nitro-20(S)-Camptothecin (9NO₂) and 9-Amino-20(S)-Camptothecin (9AC) were also synthesized from CPT. CPT and derivatives thereof have to be extensively purified prior to administering for use in the present invention because: (1) the natural product contains several other components which have a large degree of toxicity for example, in mice, and (2) FDA regulations require such purifying for any drug or compound to be used as a medicine. Methods of purification known by those skilled in the art can be used, e.g. - dissolving the CPT in a suitable solvent such as chloroform or methylene chloride and then adsorbing onto a column containing silica gel and then carrying out elution of the adsorbed materials by increasing the polarity of the eluant by adding, e.g., methanol.

The purity of the compound can be tested by high performance liquid chromatography (HPLC) and thin layer chromatography (TLC) and other appropriate methods known in the art. The compound can also be completely characterized using infrared (IR), ultraviolet (UV) and nuclear magnetic resonance (NMR) spectroscopy and elemental analysis.

Furthermore, the CPT and derivatives thereof used in the present invention are water-insoluble and not administered in the chemically and physically different sodium salt form. This difference can easily be observed by looking at their elemental analysis, NMR, UV and IR spectra and also by their different physical behavior in HPLC and TLC experiments.

CPT and derivatives thereof of the closed lactone ring form are used and are administered intramuscularly or orally, and in such cases it was possible to obtain total remissions of a vast spectrum of human cancers without the toxicity observed previously with CPT Na+. The derivatives of CPT for use in the present invention are 9NO₂, and 9AC. A mixture of CPT and derivatives thereof can also be used in the present invention.

Other related derivatives can also be used in conjunction with the method of the present invention. Examples include dimethylaminomethyl-10-hydroxy-20(S)-CPT (topotecan), 7-ethyl-10-[4-(1-piperdino)-1-piperdino]-carbonyloxy-CPT (CPT-11), 7-ethyl-10-hydroxy-20(S)-CPT, 9-amino-20(S)-CPT, 9-nitro-20(S)-CPT, 10,11-methylenedioxy-20(S)-CPT, 9-chloro-20(S)-CPT, 9-bromo-20(S)-CPT, 9-hydroxy-20(S)-CPT, 11-hydroxy-20(S)-CPT, and 10-hydroxy-20(S)-CPT.

In the studies, the water-soluble derivatives of these compounds proved to be ineffective as anticancer agents and very toxic to mammals. Even the closed lactone ring forms of CPT and derivatives thereof showed a great difference in activity, depending on their route of administration. In the studies, the intramuscular and oral routes were unexpectedly superior to other routes of delivery.

As used herein, the term "malignant tumor" is intended to encompass all forms of human carcinomas, sarcomas and melanomas which occur in the poorly differentiated, moderately differentiated, and well differentiated forms.

In treating or retarding malignant tumors in mammals, the aforedescribed camptothecin compounds are administered intramuscularly, or orally, using commonly known methods, for example, gelatin capsules for oral administration, as well as novel, superior formulations, such as microsuspensions in lipid and in lipid-like emulsions (e.g. - Intralipid 20, cottonseed oil and peanut oil) for intramuscular administration.

As used herein, an "effective amount" of CPT and derivatives thereof described above is intended to mean that amount of the compound which will inhibit the growth of, or retard cancer, or kill malignant cells, and cause the regression and palliation of malignant tumors, i.e., reduce the volume or size of such tumors or eliminate the tumor entirely.

With mammals, including humans, the effective amounts can be administered on the basis of body surface area. The interrelationship of dosages for animals of various sizes, . species and humans (based on mg/M² of body surface) is described by E.J. Freireich et al., Cancer Chemother. Rep., 50(4):219 (1966). Body surface area may be approximately determined from the height and weight of an individual (see, e.g., Scientific Tables, Geigy Pharmaceuticals, Ardsley, N.Y. pp. 537-538 (1970). An effective amount of the camptothecin compounds in the present invention can range from about 12.5 mg/m² of body surface per day to about 31.3 mg/m² of body surface per day.

The preferred effective amounts or dosages of CPT and/or derivatives thereof in mice are about 1 to about 4 mg CPT/kg of body weight twice a week for an intramuscular route and about 0.75 to about 1.5 mg CPT/kg/day for the oral route. For all of the administering routes, the exact timing of administration of the dosages can be varied to achieve optimal results. Generally, when using Intralipid 20 as the carrier for the CPT, the actual dosage of CPT reaching the patient will be less. This is due to some loss of CPT on the walls of the syringes, needles and preparation vessels, which is prevalent with the Intralipid 20 suspension. When a carrier, such as cottonseed oil is used, this above-described loss is not so prevalent because CPT does not adhere as much to the surfaces of syringes, etc... For instance and preferable, it has been found that generally about 2.5 mg CPT/kg of body weight twice per week using cottonseed oil, administered by an intramuscular route, will deliver the same amount to the patient as 4.0 mg CPT/kg of body weight twice per week using Intralipid 20 as a carrier. Generally, about 1 mg to about 4 mg of CPT and/or derivatives thereof is added to about 0.1 ml to about 1 ml of carrier. Levels of both CPT and 9NO₂ were well tolerated by mice in the examples set forth below without weight loss or other signs of toxicity. These dosages have been administered for up to six months continuously without any ill effect.

Another important feature of the method relates to the relatively low or no apparent overall toxicity of the camptothecin compounds administered in accordance herein. Overall toxicity can be judged using various criteria. For example, loss of body weight in a subject over 10% of the initially recorded body weight (i.e., before treatment) can be considered as one sign of toxicity. In addition, loss of overall mobility and activity and signs of diarrhea or cystitis in a subject can also be interpreted as evidence of toxicity. In one of the examples which follow, the overall toxicity of the camptothecin compounds of the present invention was evaluated. For example, CPT, when administered twice per week in amounts of 4 mg/kg in Intralipid 20 or 2.5 mg/kg in cottonseed oil, were found to be therapeutically effective (resulting in complete reduction of subcutaneous tumors) with no overall toxicity as described above. This was also the case when administering for more than 35 weeks the enormous total dose of 280 mg/kg.

Further, using 0.1 to 1.0 ml of cottonseed oil, in which CPT or derivatives were suspended, up to a quantity of 1 to 4 mg/kg were injected into the cavity of the stomach through a 27-gauge needle once a day for as long as desired (mice have tolerated more than 40 consecutive, daily injections) with no ill-effects.

The compounds used in the present invention may be administered in combination with pharmaceutically acceptable carriers or diluents, such as Intralipid 10 or 20 or natural oils, or other suitable emulsifiers for lipophilic compounds.

The present invention will be further clarified by the following example, which is intended to be purely exemplary of the present invention.

### Example 1

Swiss nude mice of the NIH high fertility strain were bred and maintained pathogen-free in a laboratory. (Giovanella, B.C. and Stehlin, J.S. "Heterotransplantation of human malignant tumors in 'nude' thymusless mice. I. Breeding and maintenance of 'nude' mice." J. Natl. Cancer Inst. 51: 615-619; 1973.)

Human malignant carcinomas of the pancreas were heterotransplanted directly from a patient into the nude mice and passaged serially. For the experiments, the tumor tissue was finely minced in complete MEM medium and 0.5 ml of a 10% v/v suspension was inoculated subcutaneously on the upper back of groups of 10-30 mice. When the tumors became palpable and measurable in all the animals, they were divided into groups of 4-8 and treated with the desired dose of the drug in experiment or with the vehicle only for the controls.

9NO₂ (also referred to as 9NC) and 9AC were synthesized (Wani, M.C., Nicholas, A.W., Wall, M.E., "Plant Tumor Agents. 23. Synthesis and antileukemic Activity of Camptothecin analogues, J. Med. Chem., 2358-2363, 1986) from CPT obtained from the Institute of Materia Medica, Academia Sinica, Shanghai, China. The derivatives were purified by chromatography and analyzed. A typical sample preparation for IM-injection or oral administration using Intralipid 20 or cottonseed oil for example includes the dispersion of the test compound by sonication (3 pulses for 30 seconds each) in Intralipid 20 or cottonseed oil at the standard concentration of 1 mg/ml using an Ultrasonic Processor, Model GE600 by Sonics and Materials, Inc., CT. Intramuscular injections were performed through a 27-gauge needle into the deep muscles of the posterior legs of the mice twice a week. The animals received up to 70 such injections consecutively without suffering ill effects except for some local fibrosis. Intravenous injections, using Intralipid 20 as the suspending agent, were performed through a 30-gauge needle in one of the tail veins. No pulmonary embolism was observed after over 200 injections.

Oral administration was achieved by mixing the required amounts of drugs in Intralipid 20 or cottonseed oil with a previously autoclaved dietary supplement composed of whole wheat bread saturated with whole milk. The supplement containing the drug was then thoroughly mixed to form a paste. Mice were fed 1 g of dietary supplement containing the required dose of the drugs once a day, and then 5 g of autoclaved mouse feed. (At this regime, the animals initially lose about 4 g out of 30 g of body weight, regain 2 g within a few days, and stabilize at this level). This regime can be carried on indefinitely. Actually, the mice fed in this manner (a slightly lower maximum caloric intake) were healthier and longer-lived than mice fed ad lib (Giovanella, B.C., Shepard, R.C., Stehlin, J.S., Venditti, J.M. and Abbott, B.J. "Calorie restriction: Effect on growth of human tumors heterotransplanted in nude mice." J. Natl. Cancer Inst. 68: 249-257; 1982).

Figures 1 and 2 show the results obtained for LOV pancreatic cancer. Seven nude mice were treated intramuscularly with 2.5 mg/Kg body weight of 20(S)-CPT in cottonseed oil twice a week until day 100. Six additional nude mice were used as a control (cottonseed oil only). After 125 days, all mice receiving 20(S)-CPT survived as compared to only two of the six mice in the control group.

It is clear from these studies, that CPT and its derivatives with the closed lactone ring have been demonstrated to possess an astonishing level of anticancer activity. This applies both to the spectrum of tumors covered and to the quality of the responses. The method of the present invention has been able to block growth completely and to totally regress human xenografts of carcinomas (pancreas). This has been accomplished without any observable toxicity. Many of the mammals which have been treated continuously for six months have shown no ill effects nor regrowth of the tumor they once carried.

Other embodiments of the present invention will be apparent to those skilled in the art from consideration of the specification and practice of the present invention disclosed herein. It is intended that the specification and examples be considered as exemplary only.

## Claims

1. Use of an agent which is 20(S)-camptothecin, 9-Nitro-20(S)-camptothecin, 9-Amino-20(S)-camptothecin, or a mixture thereof, in the manufacture of a medicament for the treatment of pancreatic cancer in a human wherein said agent is for oral or intramuscular administration of an effective amount of said agent.

2. Use according to claim 1, wherein said agent is 9-Nitro-20(S)-camptothecin.

3. Use according to claim 1, wherein said agent is 9-Amino-20(S)-camptothecin.

4. Use according to any one of claims 1 to 3, wherein said agent is to be administered orally.

5. Use according to any one of claims 1 to 3, wherein said agent is to be administered intramuscularly.

## Patentansprüche

1. Verwendung eines Mittels, welches 20(S)-Camptothecin, 9-Nitro-20(S)-Camptothecin, 9-Amino-20(S)-Camptothecin oder ein Gemisch davon ist, in der Herstellung eines Medikaments zur Behandlung von Pankreaskrebs beim Menschen, wobei das Mittel zur oralen oder intramuskulären Verabreichung einer wirksamen Menge des Mittels bestimmt ist.

2. Verwendung nach Anspruch 1, wobei das Mittel 9-Nitro-20(S)-Camptothecin ist.

3. Verwendung nach Anspruch 1, wobei das Mittel 9-Amino-20(S)-Camptothecin ist.

4. Verwendung nach einem der Ansprüche 1 bis 3, wobei das Mittel oral zu verabreichend ist.

5. Verwendung nach einem der Ansprüche 1 bis 3, wobei das Mittel intramuskulär zu verabreichend ist.

## Revendications

1. Utilisation d'un agent qui est la 20 (S) - camptothecine, la 9 - nitro - 20 (S) - camptothecine, la 9 - amino - 20 (S) - camptothecine, ou un mélange de celles-ci, dans la préparation d'un médicament pour le traitement du cancer du pancréas chez un humain, dans laquelle ledit agent est destiné à l'administration orale ou intra-musculaire d'une quantité efficace dudit agent.

2. Utilisation selon la revendication 1, dans laquelle ledit agent est la 9 - nitro - 20 (S) -camptothecine.

3. Utilisation selon la revendication 1, dans laquelle ledit agent est la 9 - amino - 20 (S) -camptothecine.

4. Utilisation selon l'une quelconque des revendications 1 à 3, dans laquelle ledit agent est destiné à être administré par voie orale.

5. Utilisation selon l'une quelconque des revendications 1 à 3, dans laquelle ledit agent est destiné à être administré par voie intra-musculaire.
